# EUROPEAN PATENT APPLICATION

(11) **EP 1 002 515 A1**
(43) Date of publication of application: **24.05.2000**
(21) Application number: 99923915.5
(22) Date of filing: 04.06.1999
(51) Int. Cl.: A61K 7/00, A61K 7/48

(54) **SKIN COMPOSITIONS FOR EXTERNAL USE**

(30) Priority: 05.06.1998 JP 17386798
(71) Applicant: SHISEIDO COMPANY LIMITED, Chuo-ku, Tokyo 104-8010 (JP)
(72) Inventor: TANAKA, Naomi, Shiseido Res. Cent.(1), Yokohama-shi, Kanagawa 223-8553 (JP); YAGI, Eiichiro, Shiseido Res. Cent.(1), Yokohama-shi, Kanagawa 223-8553 (JP); NAGANUMA, Masako, Shiseido Res. Cent.(1), Yokohama-shi, Kanagawa 223-8553 (JP)
(74) Representative: Santarelli, Marc
(86) International application number: JP9903004
(87) International publication number: WO9963950

(57) **Abstract**

Skin compositions for external use which contain a first component selected from the group consisting of L-ascorbic acid and its derivatives, placenta extract, kojic acid and its derivatives, azelaic acid and its derivatives, glucosamine and its derivatives, hydroquinone glycosides and derivatives thereof, tranexamic acid and its derivatives and salicylic acid and its derivatives, and a second component selected from Lempuyang and solvent extracts thereof.

## Description

### TECHNICAL FIELD

The present invention relates to an external skin treatment composition having remarkably improved skin whitening effect, superior moisture retention effect, and superior stability and safety.

### BACKGROUND ART

Pigmentation problems such as skin pigmentation, freckles arise due to rampant production of melanin in the epidermal pigment cells and excessive deposition of melanin in the epidermis triggered by hormonal abnormalities or stimulation by UV rays. To prevent pigmentation and freckles, there are the method of administering large dosages of substances suppressing the production of melanin, for example, L-ascorbic acid, the method of injecting glutathione etc., or the method of forming kojic acid, cysteine, or the like into an ointment, cream, lotion, or the like and coating it locally.

However, most of these have problems in the stability, safety, odor, or other areas. Further, the anticipated effects are weak and are still not satisfactory. Further, when an external skin treatment composition having a moisture retention effect is sought, it is necessary to formulate a separate moisture retainer etc. In this case, there was the disadvantage that the skin became sticky and the feeling in use was impaired.

### DISCLOSURE OF THE INVENTION

Accordingly, the object of the present invention is to provide an external skin treatment composition having both a superior whitening effect and moisture retention effect.

In accordance with the present invention, there is provided an external skin treatment composition comprising at least one first component selected from the group consisting of L-ascorbic acid and its derivatives, placental extracts, kojic acid and its derivatives, azelaic acid and its derivatives, glucosamine and its derivatives, hydroquinone glycosides and their derivatives, tranexamic acids and their derivatives, and salicylic acid and its derivatives and a second component of *Zingiber aromaticum (Zingiberaceae)* or its solvent extract.

### BEST MODE FOR CARRYING OUT THE INVENTION

The present inventors found that, by using at least one first component selected from the group consisting of L-ascorbic acid and its derivatives, placental extracts, kojic acid and its derivatives, azelaic acid and its derivatives, glucosamine and its derivatives, hydroquinone glycosides and their derivatives, tranexamic acids and their derivatives, and salicylic acid and its derivatives in combination with a second component of *Zingiber aromaticum (Zingiberaceae)* or its solvent extract, a synergistic whitening effect can be obtained, when compared with the case of use of these alone, and also a moisture retention effect, and the stability can be improved, whereby the present invention can be completed.

The present invention will now be explained in detail.

The L-ascorbic acid usable in the present invention is generally called vitamin C and exhibits an inhibitory action on the tyrosinase reaction, the controlling step of the melanin action, due to the strong reducing action and exhibits a reducing action on melanin.

Further, as derivatives of L-ascorbic acid, for example, L-ascorbyl monoalkyl esters such as L-ascorbyl monostearate, L-ascorbyl monopalmitate, and L-ascorbyl monooleate; L-ascorbyl monoester derivatives such as L-ascorbyl monophosphate esters and L-ascorbyl-2-sulfate esters; dialkyl esters such as L-ascorbyl distearate, L-ascorbyl dipalmitate, and L-ascorbyl dioleate; L-ascorbyl diesters such as L-ascorbyl diphosphate esters; trialkyl esters such as L-ascorbyl tristearate, L-ascorbyl tripalmitate, and L-ascorbyl triooleate; ascorbyl triesters such as L-ascorbyl triphosphate esters, etc. may be mentioned.

Particularly preferable as the L-ascorbic acid and its derivatives are L-ascorbic acid, L-ascorbyl phosphate esters, L-ascorbyl-2-sulfate esters, or their salts.

As the kojic acid derivatives usable in the present invention, for example a kojic acid ester such as a kojic acid alkyl ester or a kojic acid ether such as a kojic acid alkyl ether may be mentioned.

As the azelaic acid derivatives usable in the present invention, for example an azelaic acid monoester such as an azelaic acid monoalkyl ester and an azelaic acid diester such as an azelaic acid dialkyl ester may be mentioned.

As the glucosamine derivatives usable in the present invention, for example, glucosamine esters such as acetyl glucosamine or glucosamine ethers such as glucosamine methylether may be mentioned.

As the glycoside of the hydroquinone usable in the present invention, for example, hexose glycosides such as hydroquinone α-D-glucose, hydroquinone β-D-glucose, hydroquinone α-L-glucose, hydroquinone β-L-glucose, hydroquinone α-D-galactose, hydroquinone β-D-galactose, hydroquinone α-L-galactose, or hydroquinone β-L-galactose; pentose glycosides such as hydroquinone α-D-ripose, hydroquinone β-D-ripose, hydroquinone α-L-ripose, hydroquinone β-L-ripose, hydroquinone α-D-arabinose, hydroquinone β-D-arabinose, hydroquinone α-L-arabinose, and hydroquinone β-L-arabinose; aminosugar glycosides such as hydroquinone α-D-glucosamine, hydroquinone β-D-glucosamine, hydroquinone α-L-glucosamine, hydroquinone β-L-glucosamine, hydroquinone α-D-galactosamine, hydroquinone β-D-galactosamine, hydroquinone α-L-galactosamine, and hydroquinone β-L-galactosamine; and uronic acid glycosides such as hydroquinone α-D-gluconic acid, hydroquinone β-D-gluconic acid, hydroquinone α-L-gluconic acid, hydroquinone β-L-gluconic acid, hydroquinone α-D-galucturonic acid, hydroquinone β-D-galucturonic acid, hydroquinone α-L-galuturonic acid, hydroquinone β-L-galucturonic acid, etc.

Further, as their derivatives, an ester such as an acetylate, an ether such as a methylate, etc. may be mentioned, but judging from the whitening effect, the ease of acquisition, the shelf life, etc., use of hydroquinone β-D-glucose (general name: arbutin, hereinafter called "arbutin") is preferable.

As the derivatives of tranexamic acids usable in the present invention, dimers of tranexamic acids (trans-4-(trans-4-aminomethylcyclohexanecarbonyl) aminomethylcyclohexane carboxylate hydrochloride), esters of tranexamic acids and hydroxyquinone (trans-4-aminomethylcyclohexane carboxylate-4'-hydroxyphenylester), esters of tranexamic acids and gentisic acid (2-(trans-4-aminomethylcyclohexylcarbonyloxy)-5-hydroxybenzoic acid and its salts), amides of tranexamic acids (trans-4-aminomethylcyclohexanecarboxylate methylamides and their salts, trans-4-acetylaminomethylcyclohexane carboxylic acids and their salts, trans-4-(p-methoxybenzoyl)aminomethylcyclohexane carboxylic acids and their salts, trans-4-guanidinomethylcyclohexane carboxylic acids and their salts etc.) etc.

As derivatives of salicylic acid usable in the present invention, 3-methoxysalicylic acid and its salts, 4-methoxysalicylic acid and its salts, 5-methoxysalicylic acid and its salts, etc. may be mentioned.

In the practice of the present invention, one or more of these first component is suitably selected and formulated, but particularly preferable among these are L-ascorbic acid or its derivatives such as L-ascorbic acid, L-ascorbyl phosphate esters, and L-ascorbyl-2-sulfate esters and hydroquinone β-D-glucose.

The content of the one or more types of components selected from the group consisting of L-ascorbic acid and its derivatives, placental extracts, kojic acid and its derivatives, azelaic acid and its derivatives, glucosamine and its derivatives, hydroquinone glycoside and its derivatives, tranexamic acids and their derivatives, and salicylic acid and its derivatives is not particularly limited, but in general is 0.001 to 20.0% by weight based upon the total weight of the external skin treatment composition, preferably 0.01 to 10.0% by weight, particularly preferably 0.1 to 7.0% by weight. When the content is less than 0.001% by weight, the whitening effect of the external skin treatment agent tends to become poor. Conversely, even when formulated in an amount of more than 20.0% by weight, no substantial increase in the whitening effect can be expected and formulation into the external skin treatment agent also tends to become difficult.

The *Zingiber aromaticum (Zingiberaceae)*, another essential component contained in the external skin treatment composition according to the present invention, is a perennial produced in Indonesia which is occasionally cultivated for medicinal use and grows in the wild in various areas. In the present invention, the root and stem or entirety of the plant is dried, then pulverized to form a powder or is immersed or heated and refluxed together with an extraction solvent, then filtered and the filtrate concentrated or the solvent removed, but it is preferable to use it as an extract from the standpoint of usability. This extraction solvent may be any solvent which is normally used for extraction. In particular, water, alcohols such as methanol, ethanol, propylene glycol, and 1,3-butylene glycol, aqueous alcohols, urea-containing alcohols, organic solvents such as acetone, ethyl acetate esters may be used alone or in any combination thereof.

The present inventors already found that *Zingiber aromaticum (Zingiberaceae)* and its solvent extracts have a whitening action (Japanese Unexamined Patent Publication (Kokai) No. 9-71522), but newly found that, by the use of a conventionally known whitening agent such as L-ascorbic acid and its derivatives, placental extracts, or kojic acid and its derivatives in combination with *Zingiber aromaticum (Zingiberaceae)* or its solvent extract, the whitening effect can be synergistically improved the problems in the stability etc. of conventionally known whitening agents are solved, and a moisture retention effect can also be given.

The content of the *Zingiber aromaticum (Zingiberaceae)* or its solvent extract is not particularly limited, but generally is, in terms of dry solid content, 0.0001 to 20.0% by weight, preferably 0.01 to 10.0% by weight, based upon the total weight of the external skin treatment composition. When the content is less than 0.0001% by weight, the whitening effect and moisture retention effect of the external skin treatment composition are insufficient and the effect of suppressing the skin irritation by the external skin treatment composition tends to be poor. Conversely, even when the formulation amount is more than 20.0% by weight, no substantial increase in the effect can be expected and the formulation into the external skin treatment composition tends to become difficult.

Further, a UV protector may be further formulated into the external skin treatment composition of the present invention so as to further improve the whitening effect.

The UV protector used in the present invention includes both a "UV absorber" for absorbing UV rays physiochemically and a "UV blocker" for scattering and reflecting UV rays by physical means. These UV absorbers and UV blockers may be used alone or in any combinations thereof.

As the UV absorber, benzoate-based UV absorbers such as para-aminobenzoic acid (hereinafter referred to as "PABA"), PABA monoglycerin ester, N,N-dipropoxy PABA-ethyl ester, N,N-diethoxy PABA ethyl ester, N,N-dimethyl PABA-ethyl ester, N,N-dimethyl PABA-butyl ester, and N,N-dimethyl PABA-amyl ester; anthranilic acid-based UV absorbers such as homomenthyl-N-acethyl anthranilate etc.; salicylate-based UV absorbers such as amylsalicylate, menthylsalicylate, homomenthylsalicylate, octylsalicylate, phenylsalicylate, benzylsalicylate, and p-isopropanol phenylsalicylate; cinnamate-based UV absorbers such as octylcinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, glycerylmono-2-ethylhexanoyl-diparamethoxycinnamate, and 3,4,5-trimethylcinnamate 3-methyl-4-[methylbis(trimethylsiloxy)silyl]butyl; benzophenone-based UV absorbers such as 2,4-dihydroxybenzophenone, 2,2'-dihydroxy-4-methoxybenzophenone, 2,2'-dihydroxy-4,4'-dimethoxybenzophenone, 2,2,4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid, 4-phenylbenzophenone, 2-ethylhexyl-4'-phenyl-benzophenone 2-carboxylate, 2-hydroxy-4-n-octoxybenzophenone, and 4-hydroxy-3-carboxybenzophenone; 3-(4'-methylbenzylidene)d,1-camphor, 3-benzylidene d,1-camphor, urocanic acid, urocanate ethyl esters, 2-phenyl-5-methylbenzoxanole, 2,2-hydroxy-5-methylphenylbenzotriazole, 2-(2'-hydroxy-5'-t-octylphenyl)benzotriazole, 2-(2'-hydroxy-5'-methylphenylbenzotriazole, dibenzalazine, dianisoylmethane, 4-tert-butyl-4'-methoxydibenzoylmethane, 5-(3,3-dimethyl-2-norbornylidene)-3-pentan 2-one, etc. may be mentioned. Particularly preferably 4-tert-butyl-4'-methoxydibenzoylmethane, octyl-p-methoxycinnamate, 2-hydroxy-4-methoxybenzophenone, 2-hydroxy-4-methoxybenzophenone-5-sulfonate, etc. may be mentioned.

Further, as the UV blockers, titanium dioxide (TiO₂), talc (MgSiO₂), carmine (FeO₂), bentonite, kaolin, zinc oxide (ZnO), etc. may be mentioned.

When blending in such a UV protector, the amount blended normally is preferably 0.01 to 30.0% by weight of the total weight of the external skin treatment composition, more preferably 0.1 to 20.0% by weight.

The external skin treatment composition of the present invention may have suitably formulated therein, in addition to the above essential components, other components normally used for external skin treatment compositions such as cosmetics or pharmaceuticals, for example, oils, moisturizers, anti-oxidants, surfactants, preservatives, moisture retention agents, perfumes, water, alcohols, thickeners, etc., if desired.

The carrier of the external skin treatment composition according to the present invention may be of any type. For example, it may be made solubilized type such as cosmetic water, emulsified type such as emulsion, cream, any other type such as ointment, dispersion.

### EXAMPLES

The present invention will now be explained in further detail with reference to Examples, but the technical scope of the present invention is by no means limited to these Examples. Note that the amounts in the following Examples are % by weight.

### Examples 1 to 8 and Comparative Examples 1 to 9

| (Alcohol Phase) | |
|---|---|
| 95% Ethanol | 25.0 wt% |
| Polyoxyethylene(25 mol)hydrogenated castor oil ether | 2.0 |
| 2-Hydroxy-4-methoxybenzophenone-5-sulfonate | 3.0 |
| Preservative and antioxidant | q.s. |
| Perfume | q.s. |
| Medicine (see Table I or Table II) (wt%) | |

| (Aqueous Phase) | |
|---|---|
| Glycerol | 2.0 |
| Propylene glycol | 1.0 |
| Ion exchanged water | Bal. |

### Preparation Method

The aqueous phase and alcohol phase were prepared, and then solubilized.

The lotions of Examples 1 to 8 and Comparative Examples 1 to 9 obtained were used to judge the whitening effect and moisture retention effect from a test of use such as the whitening effect on the skin, the elimination of pigmentation and freckles, and the like due to cumulative coating. The test method and the evaluation method are shown below. The results are also shown in Table I and Table II.

### (1) Whitening Effect

### (Test Method)

A group of 20 test subjects suffering from dark complexion, pigmentation, freckles, etc. were asked to coat sample lotions of Examples 1 to 8 and Comparative Examples 1 to 9 every day on the face in the morning and evening for three months. After three months, the whitening effect was investigated. The extent of the dark complexion, pigmentation, and freckles before and after the start was evaluated in seven ranks:

### (Evaluation Criteria)

1: No dark complexion, pigmentation, and freckles.
2: Minor degree of dark complexion, pigmentation, and freckles.
3: Light degree of dark complexion, pigmentation, and freckles.
4: Medium light degree of dark complexion, pigmentation, and freckles.
5: Medium degree of dark complexion, pigmentation, and freckles.
6: Medium high degree of dark complexion, pigmentation, and freckles.
7: High degree of dark complexion, pigmentation, and freckles.

### (Evaluation)

++: At least 80% of test subjects reported improvement of at least 2 ranks (valid ratio)
+: At least 50% to less than 80% of test subjects reported improvement of at least 2 ranks (valid ratio)
±: At least 30% to less than 50% of test subjects reported improvement of at least 2 ranks (valid ratio)
-: Less than 30% of test subjects reported improvement of at least 2 ranks (valid ratio)

### (2) Moisture Retention Effect

Twenty healthy subjects subjected to different amounts of UV irradiation in advance and measured for minimum erythema dose (MED) were irradiated at their stomach portions to 1.44 MED using two lamps, an FL40SE lamp and a BLB lamp (made by Toshiba). 2 x 2 cm portions irradiated by UV rays were determined as portions for coating the samples of Examples 1 to 8 and Comparative Examples 1 to 9, portions for coating samples of the same formulations as the Examples and Comparative Examples but without the medicines, and portions not coated with anything (control) and the treatment continued for one week. After the one week, the skin conductance was measured in a constant temperature, constant humidity room (using SKICON-200 made by IBS Co.) and the corneal moisture content was found.

The values of the corneal moisture content of the portions coated with the samples of the Examples and Comparative Examples indexed to the corneal moisture content of the portions coated with samples not containing any medicines as "1" and the average values of the values of the corneal moisture content of the portions coated with the samples of the Examples indexed to the corneal moisture content of the control as "1" are shown in Table I and Table II.

**Table I**

| Example | 1 | 2 | 3 | 4 | 5 | 6 | 7 | 8 |
|---|---|---|---|---|---|---|---|---|
| L-Ascorbic acid phosphoric acid ester magnesium salt | 1.0 | - | - | - | - | - | - | - |
| Placental extract | - | 1.0 | - | - | - | - | - | - |
| Arbutin | - | - | 1.0 | - | - | - | - | - |
| Kojic acid | - | - | - | 1.0 | - | - | - | - |
| Azelaic acid | - | - | - | - | 1.0 | - | - | - |
| Glucosamine | - | - | - | - | - | 1.0 | - | - |
| Tranexanic acid | - | - | - | - | - | - | 1.0 | - |
| Salicylic acid | - | - | - | - | - | - | - | 1.0 |
| *Zingiber aromaticum (Zingiberaceae)* 80% aqueous ethanol solution reflux extract (as dry solid) | 1.0 | 1.0 | 1.0 | 1.0 | 1.0 | 1.0 | 1.0 | 1.0 |
| Whitening effect | ++ | + | ++ | + | + | + | + | + |

| Moisture retention effect | | | | | | | | |
|---|---|---|---|---|---|---|---|---|
| Sample with no medicine | 1.4 | 1.2 | 1.4 | 1.2 | 1.2 | 1.2 | 1.3 | 1.3 |
| Control | 2 | 1.3 | 2 | 1.3 | 1.3 | 1.3 | 1.5 | 1.5 |

**Table II**

| Comparative Example | 1 | 2 | 3 | 4 | 5 | 6 | 7 | 8 | 9 |
|---|---|---|---|---|---|---|---|---|---|
| L-Ascorbic acid phosphoric acid ester magnesium salt | 2.0 | - | - | - | - | - | - | - | - |
| Placental extract | - | 2.0 | - | - | - | - | - | - | - |
| Arbutin | - | - | 2.0 | - | - | - | - | - | - |
| Kojic acid | - | - | - | 2.0 | - | - | - | - | - |
| Azelaic acid | - | - | - | - | 2.0 | - | - | - | - |
| Glucosamine | - | - | - | - | - | 2.0 | - | - | - |
| Tranexamic acid | - | - | - | - | - | - | 2.0 | - | - |
| Salicylic acid | - | - | - | - | - | - | - | 2.0 | - |
| *Zingiber aromaticum (Zingriberaceae)* 80% aqueous ethanol solution reflux extract (as dry solid) | - | - | - | - | - | - | - | - | 2.0 |
| Whitening effect | + | - | + | - | - | - | ± | ± | + |

| Moisture retention effect | | | | | | | | | |
|---|---|---|---|---|---|---|---|---|---|
| Sample with no | 1 | 1 | 1 | 1 | 1 | 1 | 1 | 1 | 1.1 |
| medicine | | | | | | | | | |

As clear from the results of Table I and Table II, it was observed that compared with the Comparative Examples, the Examples of the invention have a synergistic skin whitening effect and are provided with both the effects of whitening and moisture retention.

### Example 9. Vanishing Cream

| | |
|---|---|
| Stearic acid | 6.0 wt% |
| Sorbitan monostearate ester | 2.0 |
| Polyoxyethylene(20 mol)sorbitan monostearate ester | 1.5 |
| Arbutin | 7.0 |
| Sodium bisulfite | 0.03 |
| Propylene glycol | 10.0 |
| *Zingiber aromaticum (Zingiberaceae)* 80% aqueous ethanol solution (as dry solid) | 1.0 |
| Preservative and antioxidant | q.s. |
| Perfume | q.s. |
| Ion exchanged water | Bal. |

### Preparation Method

The *Zingiber aromaticum (Zingiberaceae)* 80% aqueous ethanol solution reflux extract, the arbutin, and the propylene glycol were added to the ion exchanged water, then the mixture was heated and held at 70°C (aqueous phase). The rest of the components were mixed and heated to melt and held at 70°C (oil phase). The oil phase was added to the aqueous phase and preliminarily emulsified, emulsified homogeneously by a homogenizer, then cooled to 30°C while stirring well.

### Example 10. Neutral Cream

| | |
|---|---|
| Stearyl alcohol | 7.0 wt% |
| Stearic acid | 2.0 |
| Hydrogenated lanolin | 2.0 |
| 2-Hydroxy-4-methoxybenzophenone | 3.5 |
| Squalane | 5.0 |
| 2-Octyldodecyl alcohol | 6.0 |
| Polyoxyethylene(25 mol)cetyl alcohol ether | 3.0 |
| Glyceryl monostearate ester | 2.0 |
| Placental extract | 0.1 |
| Propylene glycol | 5.0 |
| *Zingiber aromaticum (Zingiberaceae)* 80% aqueous ethanol solution reflux extract (as dry solid) | 10.0 |
| Perfume | q.s. |
| Preservative and antioxidant | q.s. |
| Ion exchanged water | Bal. |

### Preparation Method

The *Zingiber aromaticum (Zingiberaceae)* 80% aqueous ethanol solution reflux extract, the placental extract, and the propylene glycol were added to the ion exchanged water, the mixture was then heated and held at 70°C (aqueous phase). The rest of the components were mixed and heated to melt and held at 70°C (oil phase). The oil phase was added to the aqueous phase and preliminarily emulsified, emulsified homogeneously by a homogenizer, then cooled to 30°C while stirring well.

### Example 11. Cold Cream

| | |
|---|---|
| Solid paraffin | 5.0 wt% |
| Beeswax | 10.0 |
| Vaseline | 15.0 |
| Liquid paraffin | 41.0 |
| Glyceryl monostearate ester | 2.0 |
| Polyoxyethylene(20 mol)sorbitan monolaurate ester | 2.0 |
| Kojic acid | 2.0 |
| 4-tert-Butyl-4'-methoxydibenzoyl methane | 3.5 |
| Soap powder | 0.1 |
| Borax | 0.2 |
| *Zingiber aromaticum (Zingiberaceae)* 80% aqueous ethanol solution reflux extract (as dry solid) | 0.1 |
| Ion exchanged water | Bal. |
| Perfume | q.s. |
| Preservative and antioxidant | q.s. |

### Preparation Method

The *Zingiber aromaticum (Zingiberaceae)* 80% ethanol aqueous solution reflux extract, kojic acid, soap powder, and borax were added to the ion exchanged water, the mixture was then heated to dissolve them, then held at 70°C (aqueous phase). The other components were mixed and heated to melt and the resultant mixture held at 70°C (oil phase). The oil phase was gradually added to the aqueous phase while stirring to cause a reaction. After the end of the reaction, the resultant mixture was emulsified homogeneously by a homomixer, was then cooled to 30°C while stirring well.

### Example 12. Emulsion

| | |
|---|---|
| Polyoxyethylene(20 mol)polyoxypropylene (2 mol)cetyl alcohol | 1.0 wt% |
| Octyl-p-methoxycinnamate | 3.5 |
| Silicone KF96 (20 cs) (made by Shin-Etsu Chemical) | 2.0 |
| Liquid paraffin (medium viscosity) | 3.0 |
| Propylene glycol | 5.0 |
| Arbutin | 2.0 |
| Sodium bisulfite | 0.03 |
| Glycerol | 2.0 |
| Ethanol | 15.0 |
| Carboxyvinyl polymer | 0.3 |
| Hydroxypropylcellulose | 0.1 |
| KOH | q.s. |
| Preservative | q.s. |
| *Zingiber aromaticum (Zingiberaceae)* aqueous alcohol extract (as dry solid) | 20.0 |
| Ion exchanged water | Bal. |

### Preparation Method

The *Zingiber aromaticum (Zingiberaceae)* hydrous alcohol extract and arbutin were warmed to dissolve them in ion exchanged water and ethanol, the propylene glycol and other aqueous components were then dissolved and the resultant mixture held at 70°C (aqueous phase). The other oil components were mixed and heated to melt and the resultant mixture held at 70°C (oil phase). The oil phase was added to the aqueous phase and preliminarily emulsified, was then homogeneously emulsified by a homomixer. After emulsification, the resultant emulsion was cooled to 30°C while stirring well.

### Example 13. Emulsion

| | |
|---|---|
| Polyoxyethylene(20 mol)polyoxypropylene (2 mol)cetyl alcohol | 1.0 wt% |
| Silicone KF96 (20 cs) (made by Shin-Etsu Chemical) | 2.0 |
| Liquid paraffin (medium viscosity) | 3.0 |
| Propylene glycol | 5.0 |
| Ascorbic acid | 5.0 |
| 4-tert-butyl-4'-methoxydibenzoyl methane | 3.5 |
| Glycerol | 2.0 |
| Ethanol | 15.0 |
| Carboxyvinyl polymer | 0.3 |
| Hydroxypropylcellulose | 0.1 |
| KOH | q.s. |
| Preservative | q.s. |
| *Zingiber aromaticum (Zingiberaceae)* 80% Aqueous ethanol solution reflux extract (as dry solid) | 7.0 |
| Ion exchanged water | Bal. |

### Preparation Method

The *Zingiber aromaticum (Zingiberaceae)* 80% aqueous ethanol solution reflux extract was warmed to dissolve in ion exchanged water and ethanol, the propylene glycol and other aqueous components were then dissolved and the resultant mixture held at 70°C (aqueous phase). The other oil components were mixed and heated to melt and the resultant mixture held at 70°C (oil phase). The oil phase was added to the aqueous phase and preliminarily emulsified, was then homogeneously emulsified by a homomixer. After emulsification, the resultant emulsion was cooled to 30°C while stirring well.

### Example 14. Emulsion

| | |
|---|---|
| Polyoxyethylene(20 mol)polyoxypropylene (2 mol)cetyl alcohol | 1.0 wt% |
| Silicone KF96 (20 cs) (made by Shin-Etsu chemical) | 2.0 |
| Liquid paraffin (medium viscosity) | 3.0 |
| Propylene glycol | 5.0 |
| Glycerol | 2.0 |
| 2-Hydroxy-4-methoxybenzophenone | 3.5 |
| Ethanol | 15.0 |
| Carboxyvinyl polymer | 0.3 |
| Hydroxypropylcellulose | 0.1 |
| KOH | q.s. |
| Preservative | q.s. |
| Placental extract | 5.0 |
| *Zingiber aromaticum (Zingiberaceae)* 80% Aqueous ethanol solution reflux extract (as dry solid) | 7.0 |
| Ion exchanged water | Bal. |

### Preparation Method

The *Zingiber aromaticum (Zingiberaceae)* 80% ethanol aqueous solution reflux extract and the placental extract were warmed to dissolve in ion exchanged water and ethanol, the propylene glycol and other aqueous components were then dissolved and the resultant mixture held at 70°C (aqueous phase). The other oil components were mixed and heated to melt and the resultant mixture held at 70°C (oil phase). The oil phase was added to the aqueous phase and preliminarily emulsified, then was homogeneously emulsified by a homomixer. After emulsification, the resultant emulsion was cooled to 30°C while stirring well.

### Example 15. Emulsion

| | |
|---|---|
| Polyoxyethylene(20 mol)polyoxypropylene (2 mol)cetyl alcohol | 1.0 wt% |
| Silicone KF96 (20 cs) (made by Shin-Etsu chemical) | 2.0 |
| Liquid paraffin (medium viscosity) | 3.0 |
| Propylene glycol | 5.0 |
| Glycerol | 2.0 |
| Ethanol | 15.0 |
| Carboxyvinyl polymer | 0.3 |
| Hydroxypropylcellulose | 0.1 |
| KOH | q.s. |
| Preservative | q.s. |
| Kojic acid | 3.0 |
| *Zingiber aromaticum (Zingiberaceae)* 80% Aqueous alcohol extract (as dry solid) | 3.0 |
| Ion exchanged water | Bal. |

### Preparation Method

The ion exchanged water, the *Zingiber aromaticum (Zingiberaceae)* 80% aqueous ethanol solution reflux extract, and the kojic acid were warmed to dissolve, the propylene glycol and other aqueous components were then dissolved therein and the resultant mixture held at 70°C (aqueous phase). The other oil components were mixed and heated to melt and the resultant mixture held at 70°C (oil phase). The oil phase was added to the aqueous phase and preliminarily emulsified, was then homogeneously emulsified by a homomixer. After emulsification, the resultant emulsion was cooled to 30°C while stirring well.

### Example 16. Emulsion

| | |
|---|---|
| Stearic acid | 1.5 wt% |
| Cetyl alcohol | 0.5 |
| Beeswax | 2.0 |
| Polyoxyethylene(20 mol)monooleate ester | 1.0 |
| Glyceryl monostearate ester | 1.0 |
| Ethanol | 10.0 |
| Arbutin | 20.0 |
| Sodium bisulfite | 0.03 |
| Propylene glycol | 5.0 |
| *Zingiber aromaticum (Zingiberaceae)* 80% Aqueous ethanol solution reflux extract (as dry solid) | 1.0 |
| Ion exchanged water | Bal. |
| Perfume | q.s. |
| Preservative and antioxidant | q.s. |

### Preparation Method

The *Zingiber aromaticum (Zingiberaceae)* 80% ethanol aqueous solution reflux extract, the arbutin, and the propylene glycol were added to the ion exchanged water and heated to dissolve them and the resultant mixture held at 70°C (aqueous phase). The perfume was added to the ethanol to dissolve it (alcohol phase). The other oil components were mixed and heated to melt and the resultant mixture held at 70°C (oil phase). The oil phase was added to the aqueous phase and preliminarily emulsified, was then homogeneously emulsified by a homomixer. The alcohol phase was added to this while stirring it. Next, the resultant emulsion was cooled to 30°C while stirring well.

### Example 17. Emulsion

| | |
|---|---|
| Microcrystalline wax | 1.0 wt% |
| Beeswax | 2.0 |
| Lanolin | 2.0 |
| Liquid paraffin | 20.0 |
| Squalane | 10.0 |
| Sorbitan sesquioleate ester | 4.0 |
| Polyoxyethylene(20 mol)sorbitan monooleate ester | 1.0 |
| Arbutin | 5.0 |
| Sodium bisulfite | 0.03 |
| Tranexamic acid | 5.0 |
| Propylene glycol | 7.0 |
| *Zingiber aromaticum (Zingiberaceae)* 80% Aqueous ethanol solution reflux extract (as dry solid) | 0.0001 |
| Octyl-p-methoxycinnamate | 3.5 |
| Ion exchanged water | Bal. |
| Perfume | q.s. |
| Preservative and antioxidant | q.s. |

### Preparation Method

The *Zingiber aromaticum (Zingiberaceae)* 80% ethanol aqueous solution reflux extract, the arbutin, the tranexamic acid, and the propylene glycol were added to the ion exchanged water and heated and the resultant mixture held at 70°C (aqueous phase). The other oil components were mixed and heated to melt and the resultant mixture held at 70°C (oil phase). The aqueous phase was gradually added to the oil phase while stirring the oil phase and the resultant mixture homogeneously emulsified by a homomixer. After emulsification, the resultant emulsion was cooled to 30°C while stirring well.

### Example 18. Gelly

| | |
|---|---|
| 95% Ethanol | 10.0 wt% |
| Dipropylene glycol | 15.0 |
| Polyoxyethylene(15 mol)oleyl alcohol ether | 2.0 |
| Salicylic acid | 0.5 |
| Sodium bisulfite | 0.03 |
| Ascorbic distearate | 0.5 |
| Carboxyvinyl polymer (brandname: Carbopol 941) | 1.0 |
| Potassium hydroxide | 0.15 |
| L-Arginine | 0.1 |
| *Zingiber aromaticum (Zingiberaceae)* 80% aqueous ethanol solution reflux extract (as dry solid) | 2.0 |
| Perfume | q.s. |
| Preservative | q.s. |
| Ion exchanged water | Bal. |

### Preparation Method

The *Zingiber aromaticum (Zingiberaceae)* 80% ethanol aqueous solution reflux extract, the arbutin, and the Carbopol 941 were homogeneously dissolved in the ion exchanged water. On the other hand, the dipropylene glycol, polyoxyethylene(15 mol)oleyl alcohol ether, and other components were dissolved and then added to the aqueous phase. Next, this was neutralized and thickened by the Potassium hydroxide and L-arginine.

### Example 19. Peeloff Type Pack

| (Alcohol phase) | |
|---|---|
| 95% Ethanol | 10.0 wt% |
| Polyoxyethylene(15 mol)oleyl alcohol ether | 2.0 |
| 4-tert-butyl-4'-methoxydibenzoyl methane | 3.5 |
| Preservative | q.s. |
| Perfume | q.s. |

| (Aqueous Phase) | |
|---|---|
| *Zingiber aromaticum (Zingiberaceae)* 80% aqueous ethanol solution reflux extract (as dry solid) | 3.0 |
| Azelaic acid | 1.0 |
| sodium bisulfite | 0.03 |
| Polyvinyl alcohol | 12.0 |
| Glycerol | 3.0 |
| Polyethylene glycol 1500 | 1.0 |
| Ion exchanged water | Bal. |

### Preparation Method

The aqueous phase was prepared at 80°C and cooled to 50°C. Next, the alcohol phase prepared at room temperature was added, then homogeneously mixed and allowed to cool.

### Example 20. Powder Pack

| (Alcohol phase) | |
|---|---|
| 95% Ethanol | 2.0 wt% |
| Preservative | q.s. |
| Perfume | q.s. |
| Coloring agent | q.s. |
| Ascorbic dioleate | 1.0 |

| (Aqueous Phase) | |
|---|---|
| *Zingiber aromaticum (Zingiberaceae)* 80% aqueous ethanol solution reflux extract (as dry solid) | 7.0 |
| Arbutin | 1.0 |
| Propylene glycol | 7.0 |
| Zinc white | 25.0 |
| Kaolin | 20.0 |
| Ion exchanged water | Bal. |

### Preparation Method

The aqueous phase was prepared homogeneously at room temperature. Then, the alcohol phase prepared at room temperature was added thereto, followed by homogeneously mixing.

### Example 21. Water Absorbing Ointment

| | |
|---|---|
| Vaseline | 40.0 wt% |
| Stearyl alcohol | 18.0 |
| Japan wax | 20.0 |
| Polyoxyethylene (10 mol) monooleate | 0.25 |
| Glycerin monostearate | 0.25 |
| Placental extract | 1.0 |
| Zingiber aromaticum (Zingiberaceae) 80% aqueous ethanol solution reflux extract (as dry solid) | 10.0 |
| Ion exchanged water | Bal. |

### Preparation Method

The *Zingiber aromaticum (Zingiberaceae)* 80% aqueous ethanol solution reflux extract and the placental extract were added to the ion exchanged water and the resultant mixture held at 70°C (aqueous phase). The other components were mixed and dissolved at 70°C (oil phase). The oil phase was added to the aqueous phase and homogeneously emulsified by a homomixer, then cooled.

### Example 22. Solid Foundation

| (Formulation) | |
|---|---|
| Talc | 43.1 |
| Kaolin | 15.0 |
| Sericite | 10.0 |
| Zinc white | 7.0 |
| Titanium dioxide | 3.8 |
| Yellow iron oxide | 2.9 |
| Black iron oxide | 0.2 |
| *Zingiber aromaticum (Zingiberaceae)* dried powder | 0.1 |
| Glucosamine | 1.0 |
| Squalane | 8.0 |
| Isostearic acid | 4.0 |
| Monooleate POE sorbitan | 3.0 |
| Isocetyl octanate | 2.0 |
| Preservative | q.s. |
| Perfume | q.s. |

### Preparation Method

The powder components, that is, the talc to glucosamine, were fully mixed by a blender. The oil components of the squalane to the isocetyl octanate, the preservative, and the perfume were added to this, the resultant mixture was kneaded well, then filled into a container and shaped.

The external skin treatment compositions obtained in Examples 9 to 22 were all tested for the whitening and moisture retention effect in the same way as in Examples 1 to 8, whereupon about the same extent of effects as in Examples 1 to 8 were observed.

### INDUSTRIAL APPLICABILITY

As explained above, the external skin treatment composition according to the present invention is an external skin treatment composition having remarkably improved skin whitening effect and high safety. Further, the external skin treatment composition of the present invention has a moisture retention effect, and therefore a sufficient moisture retention effect is obtained even with a little content of the moisture retention agent and it is possible to obtain a good feeling of use with no stickiness. Further, by formulating in a UV protector, the whitening effect is further improved.

## Claims

1. An external skin treatment composition comprising at least one first component selected from the group consisting of L-ascorbic acid and its derivatives, placental extracts, kojic acid and its derivatives, azelaic acid and its derivatives, glucosamine and its derivatives, hydroguinone glycosides and their derivatives, tranexamic acid and its derivatives, and salicylic acid and its derivatives and a second component of *Zingiber aromaticum (Zingiberaceae)* or its solvent extract.

2. An external skin treatment composition as claimed in claim 1, wherein the first component is L-ascorbic acid or its derivative.

3. An external skin treatment composition as claimed in claim 2, wherein the L-ascorbic acid or its derivative is L-ascorbic acid, an L-ascorbate phosphate ester, L-ascorbic acid-2-sulfate ester, or their salts.

4. An external skin treatment composition as claimed in claim 1, wherein the hydroquinone glycoside or its derivative is hydroquinone β-D-glucose.

5. An external skin treatment composition as claimed in claim 1, comprising hydroquinone β-D-glucose and *Zingiber aromaticum (Zingiberaceae)* or its solvent extract.

6. An external skin treatment composition as claimed in claim 1, wherein the content of the first component is 0.001 to 20.0% by weight based upon the total weight of the external skin treatment composition.

7. An external skin treatment composition as claimed in claim 1, wherein the content of the second component is 0.0001 to 20.0% by weight, as dry solid, based upon the total weight of the external skin treatment composition.

8. An external skin treatment composition as claimed in claim 1, which is for whitening.

9. An external skin treatment composition as claimed in claim 1, further comprising a UV protector.
